## Europäisches Patentamt
## European Patent Office
## Office européen des brevets

(19)

(11) Veröffentlichungsnummer: **0 101 402**
**B1**

(12) **EUROPÄISCHE PATENTSCHRIFT**

(45) Veröffentlichungstag der Patentschrift:
**03.09.86**

(51) Int. Cl.⁴: **C 11 D 17/00, C 11 D 3/00**

(21) Anmeldenummer: **83710044.5**

(22) Anmeldetag: **29.06.83**

(54) Reinigungs- und Desinfektionsmitteltablette für den Wasserkasten von Spültoiletten.

(30) Priorität: **07.07.82 DE 3225292**

(43) Veröffentlichungstag der Anmeldung:
**22.02.84 Patentblatt 84/8**

(45) Bekanntmachung des Hinweises auf die Patenterteilung:
**03.09.86 Patentblatt 86/36**

(84) Benannte Vertragsstaaten:
**AT BE CH DE FR GB IT LI NL SE**

(56) Entgegenhaltungen:
**EP-A-0 013 043
GB-A-2 021 143
US-A-4 308 625**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Holdt, Bernd- D., Gutenbergstrasse 435, D-4000 Düsseldorf (DE)**
Erfinder: **Menke, Ronald, Nietzschestrasse 9, D-4020 Mettmann 2 (DE)**
Erfinder: **Praus, Gerd, Höppnerstrasse 80, D-4150 Krefeld (DE)**
Erfinder: **Hasselbach, Petra, Himmelgeisterstrasse 50, D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Reinigungs- und Desinfektionsmittelstücke in Tablettenform zur Anwendung im Wasserkasten von Spültoiletten sind bekannt. Derartige Tabletten bestehen im allgemeinen aus Gemischen, die Komponenten aus der Gruppe der Tenside, Desinfektions- und Bleichmittel, Salze, Säuren, Komplexbildner, Fullstoffe, Farb- und Duftstoffe, Abspülregulatoren, Plastifikatoren, und gegebenenfalls weitere Substanzen enthalten. Beispiele solcher Tabletten finden sich in GB-A-538 857 und GB-A-2 021 143. Die Herstellung erfolgt dort durch Komprimieren einer Mischung der pulverförmigen Komponenten zu homogenen Einzelstücken. Daneben ist es üblich, bei der Herstellung derartiger Tabletten von Gemischen auszugehen, die plastifizierbar sind und in Strangform extrudiert werden können. Die Tabletten erhält man durch Schneiden des Stranges.

Es versteht sich von selbst, daß eine Kombination von Wirkstoffen, die während einer längeren Lagerzeit miteinander in Reaktion treten können oder aus anderen Gründen untereinander unvertraglich sind, in derartigen Tabletten nicht möglich ist. Es wurden daher Mehrkammer-Dispenser zum Einhängen in den Wasserkasten vorgeschlagen, welche Tabletten unterschiedlicher Zusammensetzung mit untereinander unverträglichen Wirkstoffen aufnehmen können. In derartigen Dispensern werden die entsprechenden Wirkstofflösungen getrennt hergestellt und vorrätig gehalten und erst während des Spülvorganges miteinander vereinigt. Beispiele hierfür beschreiben EP-A 13 043 und US-A-4 308 625, wobei es sich um die kombinierte Anwendung von desinfizierenden Hypochloriten und hypochloritempfindlichen Farbstoffen handelt. Die Herstellung derartiger Dispenser ist jedoch technisch aufwendig und kostspielig, und die Handhabung getrennter Mittel für den Verbraucher unbequem.

Eine andere Lösung bietet die EP-A-55 100, in der Reinigungsblöcke für die Toilette beschrieben werden, die durch Einpressen oder Einbetten einer vorgefertigten Bleichmitteltablette in eine Tensid enthaltende Masse hergestellt werden.

Die Erfindung hat zum Ziel, die geschilderten Nachteile bei der Anwendung von Reinigungs- und Desinfektiontabletten zu vermeiden.

Gegenstand der Erfindung sind daher Reinigungs- und Desinfektionsmitteltabletten für den Wasserkasten von Spultoiletten, die Substanzen aus der Gruppe Tenside, Desinfektions- und Bleichmittel, Salze, Säuren, Komplexbildner, Füllstoffe, Farb- und Duftstoffe, Abspülregulatoren und Plastifikatoren, und gegebenenfalls weitere in derartigen Formulierungen übliche Substanzen enthalten, dadurch gekennzeichnet, daß

a) die Tablette aus wenigstens zwei miteinander verbundenen plastifizierbaren Gemischen besteht,

b) wenigstens zwei der Gemische miteinander unverträgliche Substanzen einerseits aus der Gruppe aktivchlorabspaltende Substanzen, Säuren und Komplexbildner und andererseits aus der Gruppe Farbstoffe und Duftstoffe enthalten,

c) die Gemische gleiche oder ähnliche Konsistenz besitzen und

d) die Tabletten durch getrenntes Extrudieren der einzelnen Gemische, Zusammenfügen der Stränge zu einem einzigen Strang und Schneiden dieses Stranges in Stücke von 20-200g Gewicht, hergestellt wird.

Die Tabletten werden ohne Dispenser in den Wasserkasten eingelegt. Ihre Auflösegeschwindigkeit ist so bemessen, daß durchschnittlich bei jedem Spülvorgang eine ausreichende Menge an Wirkstofflösung zur Verfügung steht. Fur die Zeit zwischen zwei Spülvorgangen ist die Wirkstofflösung ausreichend stabil.

Gegenstand der Erfindung ist ferner ein Verfahren zur Herstellung der vorgenannten Tabletten, dadurch gekennzeichnet, daß man wenigstens zwei plastifizierbare Gemische, die jeweils miteinander unverträgliche Substanzen enthalten, für sich in Strangform extrudiert, die Einzelstrange zu einem einzigen Strang zusammenfügt, und diesen zu Tabletten von 20 - 200 g Gewicht schneidet. Die plastifizierbaren Gemische sollen gleiche oder ähnliche Konsistenz besitzen. Das Extrudieren und Zusammenfügen der Stränge wird zweckmäßig in einem Arbeitsgang durchgefuhrt, z.B. mit Hilfe von Doppelstrangpressen unter Vorsatz geeigneter Mundstücke.

Unter "Tabletten" sind Formstücke beliebiger Abmessungen zu verstehen, wie sie durch Schneiden eines extrudierten Stranges erhalten werden können. Insbesondere soll das Verhältnis von Durchmesser zur Dicke beliebig sein. Um eine stabile Lagerung der Tablette am Boden des Wasserkastens zu erreichen, sollte das Verhältnis von Durchmesser zur Dicke der Tablette nicht kleiner als 1, vorzugsweise 1 - 5: 1 sein.

Die Erfindung wird insbesondere angewendet auf solche Wirkstoffkombinationen, die neben einem Farbstoff ein Desinfektionsmittel auf der Basis von Aktivchlor abspaltenden Substanzen enthalten. Fast alle infragekommenden Farbstoffe, meist Grün- und Blautöne, sind mit Ausnahme weniger Triphenylmethanfarbstoffe - chlorempfindlich und verändern in Gegenwart von Hypochlorit mehr oder weniger schnell ihren Farbton oder bleichen aus. Ein Farbstoffzusatz zu Reinigungsund Desinfektionsmitteltabletten für den Wasserkasten ist aber erwünscht, da die gefärbte Spüllösung nicht nur einen Eindruck von Sauberkeit und Hygiene vermittelt, sondern durch das Ausbleiben der Farbmarkierung auch ein Signal für den Verbrauch der Tablette gibt.

Eine weitere Anwendung findet die Erfindung bei solchen Wirkstoffkombinationen, die neben einem hohen Anteil an Säuren oder Komplexbildnern zur Verhinderung von Kalk-, Rost- oder Urinsteinablagerungen empfindliche Farb- oder Duftstoffe enthalten. Insbesondere ist die Auswahl von Parfüms für die Beduftung von Toilettenreinigungsmitteln durch die starke Säureempfindlichkeit der meisten Duftstoffe normalerweise sehr begrenzt. Aber auch Komplexbildner in hohen Konzentrationen können bestimmte Farb- und Duftstoffe beeinträchtigen.

Ein besonderer Vorteil der Erfindung ist auch darin zu sehen, daß es z.B. durch Extrudieren mittels Koaxialschneckenpressen möglich ist, die Farbstoffe oder aggressive Substanzen enthaltende Komponente in den Kern der Tablette zu verlegen, was die Handhabung durch den Verbraucher erleichtert und sicherer macht, wobei auf eine besondere Umhüllung der Tabletten verzichtet werden kann.

Die folgenden Beispiele beziehen sich auf einige wichtige Ausführungsformen der erfindungsgemäßen Tabletten.

## 1. Aktivchlorabspaltende Reinigungs- und Desinfektionstablette

Dieses Beispiel betrifft eine aus zwei plastifizierbaren Gemischen zusammengesetzte Reinigungs- und Desinfektionsmitteltablette, von denen das eine eine aktivchlorabspaltende Substanz, das andere einen chlorempfindlichen Farbstoff enthält. Bei den Farbstoffen handelt es sich vorzugsweise um wasserlösliche, nicht auf Keramikflächen aufziehende Farbstoffe, z. B. Triphenylmethanfarbstoffe oder Triarylmethanfarbstoffe, wie Disulfide Blue. Die Farbstoffmarkierung ist erforderlich, um dem Verbraucher die Funktionsfähigkeit der Tablette anzuzeigen. Durch ein Ausbleiben der Farbmarkierung wird angezeigt, daß die Wirkstoffe verbraucht sind. Als chlorabspaltendes Desinfektionsmittel kommen Natriumdichloriso – cyanurat, [(Monotrichlor) -tetra-(Monokaliumdichlor)]-Penta-isocyanurat, Dichlordimethylhydantonin in Betracht.

Die Zusammensetzung einer derartigen, aus zwei plastifizierbaren Gemischen (A) und (B) bestehenden Tablette liegt in folgendem Bereich:

(A) (B)
Desinfektions- Farbkomponente
komponente
15 - 60 % 15 - 60 % anionisches Tensid
0 - 60 % 0 - 60 % anorganische Salze
- 2 - 15 % Farbstoff
5 - 50 % - aktivchlorabspaltende
Desinfektionsmittel
3 - 20 % 3 - 20 % Plastifizierungsmittel
und/oder Geruchsstoff
0 - 25 % 0 - 25 % abspülregulierende
Wirkstoffe

Bei den in den o. g. Formulierungen enthaltenen Tensiden handelt es sich um ein leicht lösliches, schaumaktives Alkylbenzolsulfonat (ABS) im Anteil von 15 - 60 %. ABS unterstützt die Haftfähigkeit der Tabletten am Boden des Wasserkastens in hohem Maße. Dadurch wird die Anwendungssicherheit auch bei starkem Wasserzulauf und Wasserablauf gewährleistet. Als abspülregulierende Zusätze dienen Kombinationen aus Stearinsäure und Kokosfettsäuremonoethanolamid im Gehalt von 2 - 15 %. Bei den konsistenzbeeinflussenden Plastifikatoren handelt es sich im wesentlichen um Parfümöl, Paraffinöl, 1,2-Propylenglykol, Silikonöl, Dibutylphthalat, Monoethylenglykol, Citrusterpene, Diethylphthalat in Anteilen von 3 - 12 Gew.-%. Dabei kann es von Vorteil sein, Plastifikatoren zu verwenden, die nicht miteinander mischbar sind, um eine Wanderung des Farbstoffes aus der gefärbten Phase in die ungefärbte Phase zu vermeiden.

Eine spezielle Formulierung hat folgende Zusammensetzung:

## Phase (A) mit Chlorträger

Dodecylbenzolsulfonat-Na mit 20 % Nacarbonat 32,0 Gew.-%
Natriumsulfat wasserfrei 10,0 Gew.-%
Natriumtripolyphosphat 13,0 Gew.-%
Natriumdichlorisocyanuratdihydrat 20,0 Gew.-%
Stearinsäure 15,0 Gew.-%
Duftstoff Pineoil 10,0 Gew.-%

## Phase (B) mit Farbstoff

Dodecylbenzolsulfonat-Na mit 2o % Nacarbonat 39,5 Gew.-%
Natriumsulfat wasserfrei 21,0 Gew.-%
Natriumtripolyphosphat 13,0 Gew.-%
Kokosfettsäuremonoethanolamid 7,0 Gew.-%
Stearinsäure 3,5 Gew.-%
Farbstoff 10,0 Gew.-%
Duftstoff Pineoil 6,0 Gew.-%

Die Herstellung einer derartigen Tablette erfolgt zweckmäßig mittels einer Koaxialstrangpresse, so daß sich das Farbstoff enthaltende Gemisch im Zentrum des ausgepreßten Stranges befindet. Der Strang wird in Stücke von etwa 50 g Gewicht geschnitten.

## 2. Reinigungsverstärkte Tablette mit hohem Säureanteil (Gemisch (A)) und Farb- und Duftstoff (Gemisch (B)) zur Verhinderung von Kalk-, Rost- und Urinsteinablagerungen

Zur Verhinderung von Kalk, Rost und/oder Urinstein ist ein relativ hoher Säureanteil wünschenswert. Gleichzeitig ist eine

Farbmarkierung mit Beduftung erforderlich. Bedingt durch die starke Säureempfindlichkeit der meisten Duftstoffe ist die Auswahl an Letzteren normalerweise äußerst begrenzt. Auch für dieses Problem stellt die erfindungsgemäße 2-Phasen-Tablette eine vorteilhafte Lösung dar, da Säure und Duftstoff getrennt in unterschiedlichen Abschnitten vorliegen.

Das nachfolgende Beispiel beschreibt eine 2-Phasen-Tablette, in deren Phase (A) sich eine Säurekomponente, wie Amidosulfonsäure, Nabisulfat, Zitronensäure oder Phosphorsäure und ein Tensidgemisch befinden, während die Phase (B) ein Tensidgemisch, einen Duftstoff und Farbstoff enthält. Der prozentuale Anteil der Säurekomponente an der Phase (A) beträgt 10 - 60 Gew.-%.

**Phase (A) mit Säurekomponente Amidosulfonsäure**

Dodecylbenzolsulfonat-Na 30,0 Gew.-%
Kokosfettsäuremonoethanolamid 7,0 Gew.-%
Stearinsäure 8,0 Gew.-%
Paraffinöl 5,0 Gew.-%
Amidosulfonsäure 50,0 Gew.-%
oder

**Phase (A) mit Säurekomponente Zitronensäure**

Dodecylbenzolsulfonat-Na 44,0 Gew.-%
Kokosfettsäuremonoethanolamid 7,0 Gew.-%
Stearinsäure 3,0 Gew.-%
Diethylphthalat 6,0 Gew.-%
Zitronensäure 40,0 Gew.-%

**Phase (B) mit säureempfindlichem Duftstoff**

Dodecylbenzolsulfonat-Na 52,0 Gew.-%
Kokosfettsäuremonoethanolamid 7,0 Gew.-%
Stearinsäure 3,0 Gew.-%
Natriumsulfat wasserfrei 10,0 Gew.-%
Natriumtripolyphosphat 10,0 Gew.-%
Farbstoff 8,0 Gew.-%
Säureempfindlicher Duftstoff 10,0 Gew.-%
Der Farbstoff ist in der Phase (A) nicht löslich, wodurch eine Wanderung des Farbstoffes in die Säurephase vermieden wird.

Die Tabletten haben zweckmäßig ein Gewicht von 50 bis 100 g.

Eine weitere, in ähnlicher Weise wirksame Tablette auf der Basis von Phosphorsäure hat folgende Zusammensetzung:

**Phase (A) mit Säurekomponente**

Dodecylbenzolsulfonat-Na 38,0 Gew.-%
Natriumsulfat wasserfrei 37,0 Gew.-%
Kokosfettsäuremonoethanolamid 7,0 Gew.-%
Stearinsäure 3,0 Gew.-%
Phosphorsäure 85 % 15,0 Gew.-%

**Phase (B) mit säureempfindlichem Duftstoff**

Dodecylbenzolsulfonat-Na 48,5 Gew.-%
Kokosfettsäuremonoethanolamid 7,0 Gew.-%
Stearinsäure 3,5 Gew.-%
Natriumsulfat wasserfrei 15,0 Gew.-%
Natriumtripolyphosphat 10,0 Gew.-%
Farbstoff 8,0 Gew.-%
Säureempfindlicher Duftstoff 8,0 Gew.-%
Die Tabletten können durch gleichzeitiges Extrudieren der Gemische (A) und (B) in einer Doppelschneckenpresse hergestellt werden, wobei das Zusammenführen der beiden Stränge und die Formgebung, z. B. rund oder rechteckig, durch ein geeignetes Mundstück erfolgt.

**3. 2-Phasen-Tablette mit verbesserter Kalk- und Rostprophylaxe auf der Basis von Komplexbildnern**

Zur Verhinderung von Kalk- und Rostablagerungen in der WC-Schüssel ist eine hohe Konzentration an Komplexbildnern notwendig. Obwohl auch diese Substanzen in bestimmten Formulierungen spezifische Farb- und Duftstoffe beeinträchtigen, gestattet die 2-Phasen-Tablette eine Kombination. Beispielsweise kann in der Phase (A) ein Komplexbildner oder Dispergator für hartwasserspezifische Ablagerungen in hoher Konzentration enthalten sein und in Phase (B) ein Tensidgemisch, Duftstoff und Farbstoff kombiniert sein. Eine bevorzugte erfindungsgemäße Formulierung mit verbesserter Kalk- und Rostprophylaxe enthält in der Phase (A) 5 - 50 % Komplexbildner oder Kalkdispergator, wie z. B, EDTA, NTA, Polycarbonsäure, Polyacrylsäure, oder Gemische aus den genannten Substanzen.

**Phase (A) mit EDTA-Komplexbilnder**

Dodecylbenzolsulfonat-Na mit 20 % Nacarbonat 43,0 Gew.-%
Natriumsulfat wasserfrei 22,0 Gew.-%
Kokosfettsäuremonoethanolami-d 7,0 Gew.-%
Stearinsäure 3,0 Gew.-%
EDTA-Natrium 15,0 Gew.-%
Chevrefeuille Super 81-2467 10,0 Gew.-%

**Phase (B) mit Farbstoff**

Dodecylbenzolsulfonat-Na mit 20 % Nacarbonat 40,0 Gew.-%
Natriumsulfat wasserfrei 21,0 Gew.-%
Natriumtripolyphosphat 13,0 Gew.-%
Kokosfettsäuremonoethanolamid 7,0 Gew.-%
Stearinsäure 3,0 Gew.-%
Farbstoff 10,0 Gew.-%
Chevrefeuille Super 81-2467 6,0 Gew.-%
Die Herstellung erfolgt durch Extrusion wie in Beispiel 2 angegeben.

**Patentansprüche**

1. Reinigungs- und Desinfektionsmitteltablette für den Wasserkasten von Spültoiletten, die Substanzen äus der Gruppe Tenside, Desinfektions- und Bleichmittel, Salze, Säuren, Komplexbildnern, Fullstoffe, Farb- und Geruchsstoffe, Abspülregulatoren und Plastifikatoren, und gegebenenfalls weitere, in derartigen Formulierungen übliche Substanzen enthält, dadurch gekennzeichnet, daß
a) die Tablette aus wenigstens zwei miteinander verbundenen plastifizierbaren Gemischen besteht,
b) wenigstens zwei der Gemische miteinander unverträgliche Substanzen einerseits aus der Gruppe aktivchlorabspaltende Substanzen, Säuren und Komplexbildner und andererseits aus der Gruppe Farbstoffe und Duftstoffe enthalten,
c) die Gemische gleiche oder ähnliche Konsistenz besitzen und
d) die Tablette durch getrenntes Extrudieren der einzelnen Gemische, Zusammenfügen der Stränge zu einem einzigen Strang und Schneiden dieses Stranges in Stücke von 20-200g Gewicht, hergestellt wird.

2. Reinigungs- und Desinfektionsmitteltablette nach Anspruch 1, die durch Extrudieren mittels Koaxialschnekkenpresse so hergestellt wird, daß ein Gemisch, das Farbstoff oder aggressive Substanzen enthält, im Kern der Tablette sitzt.

3. Reinigungs- und Desinfektionsmitteltablette nach einem der Ansprüche 1 oder 2, bei der das Verhältnis Durchmesser zu Dicke nicht kleiner als 1 ist.

4. Reinigungs- und Desinfektionsmitteltablette nach einem der Ansprüche 1 bis 3 bestehend aus zwei plastifizierbaren Gemischen A und B, die folgendermaßen zusammengesetzt sind:
A: 15 - 60 Gew.% Alkylbenzolsulfonat
0 - 60 Gew.% anorganische Salze
5 - 50 Gew.% aktivchlorabspaltende Desinfektionsmittel
3 - 20 Gew.% Plastifizierungsmittel und/oder Geruchsstoff
0 - 25 Gew.% abspülregulierende Wirkstoffe
B: 15 - 60 Gew.% Alkylbenzolsulfonat
0 - 60 Gew.% anorganische Salze
2 - 15 Gew.% Farbstoff
3 - 20 Gew.% Plastifizierungsmittel und/oder Geruchsstoff
0 - 25 Gew.% abspülregulierende Wirkstoffe.

5. Reinigungs- und Desinfektionsmitteltablette nach Anspruch 4 mit folgender Zusammensetzung:
A: 15 - 60 Gew.% Alkylbenzolsulfonat
0 - 60 Gew.% anorganische Salze
5 - 50 Gew.% aktivchlorabspaltende Desinfektionsmittel
3 - 12 Gew.% Plastifikatoren aus der Gruppe Parfümöl, Paraffinöl, 1,2-Propylenglykol, Silikonöl, Dibutylphthalat, Monoethylenglykol, Citrusterpene, Diethylphthalat und deren Mischungen,
2 - 15 Gew.% einer Kombination aus Stearinsäure und Kokosfettsäuremonoethanolamid.
B: 15 - 60 Gew.% Alkylbenzolsulfonat
0 - 60 Gew.% anorganische Salze
2 - 15 Gew.% Farbstoff
3 - 12 Gew.% Plastifikatoren aus der Gruppe Parfümöl, Paraffinöl, 1,2-Propylen-glykol, Silikonöl, Dibutylphthalat, Monoethylenglykol, Citrusterpene, Diethylphthalat und deren Mischungen,
2 - 15 Gew.% einer Kombination aus Stearinsäure und Kokosfettsäuremonoethanolamid.

6. Verfahren zur Herstellung von Reinigungs- und Desinfektionsmitteltabletten, die einem der Ansprüche 1 bis 5 entsprechen, dadurch gekennzeichnet, daß man wenigstens zwei jeweils miteinander unverträgliche Substanzen enthaltende plastifizierbare Gemische für sich in Strangform extrudiert, die Einzelstränge zu einem einzigen Strang zusammenfügt und diesen zu Tabletten von 20-200 g Gewicht schneidet.

7. Verfahren nach Anspruch 6, bei dem durch Extrudieren mittels einer Koaxialschneckenpresse eine Tablette hergestellt wird, die in ihrem Kern ein Gemisch aufweist, das Farbstoff oder aggressive Substanzen enthält.

**Claims**

1. A cleaning and disinfecting tablet for the cistern of flush toilets containing substances from the group comprising surfactants, disinfectants and bleaches, salts, acids, complexing agents, fillers, dyes and perfumes, flushing regulators and plasticizers and, optionally, other substances of the type commonly encountered in such formulations, characterized in that
a) the tablet consists of at least two interconnected plasticizable mixtures,
b) at least two of the mixtures contain incompatible substances on the one hand from the group of active chlorine donors, acids and complexing agents and, on the other hand, from the group of dyes and perfumes,
c) the mixtures have the same or similar consistency and

d) the tablet is prepared by separate extrusion of the

individual mixtures, combination of the separate strands into a single strand and cutting of this single strand into pieces weighing from 20 to 200 g.

2. A cleaning and disinfecting tablet as claimed in Claim 1 prepared by extrusion in a coaxial screw extruder in such a way that a mixture containing dye or aggressive substances is situated in the centre of the tablet.

3. A cleaning and disinfecting tablet as claimed in Claim 1 or 2 in which the ratio of diameter to thickness is no smaller than 1.

4. A cleaning and disinfecting tablet as claimed in any of Claims 1 to 3 consisting of two plasticizable mixtures A and B which have the following compositions:
A: 15 - 60% by weight alkylbenzene sulfonate
0 - 60% by weight inorganic salts
5 - 50% by weight active-chlorine yielding disinfectants
3 - 20% by weight plasticizer and/or perfume
0 - 25% by weight flushing regulators
B: 15 - 60% by weight alkylbenzene sulfonate
0 - 60% by weight inorganic salts
2 - 15% by weight dye
3 - 20% by weight plasticizer and/or perfume
0 - 25% by weight flushing regulators.

5. A cleaning and disinfecting tablet as claimed in Claim 4 having the following composition:
A: 15 - 60% by weight alkylbenzene sulfonate
0 - 60% by weight inorganic salts
5 - 50% by weight active-chlorine yielding disinfectants
3 - 12% by weight plasticizers from the group comprising perfume oil, paraffin oil, 1,2-propylene glycol, silicone oil, dibutylphthalate, monoethyleneglycol, citrus terpenes, diethylphthalate and mixtures thereof,
2 - 15% by weight of a combination of stearic acid and coconut oil fatty acid monoethanolamide.
B: 15 - 60% by weight alkylbenzene sulfonate
0 - 60% by weight inorganic salts
2 - 15% by weight dye
3 - 12% by weight plasticizers from the group comprising perfume oil, paraffin oil, 1,2-propylene glycol, silicone oil, dibutyl phthalate, monoethyleneglycol, citrus terpenes, diethylphthalate and mixtures thereof,
2 - 15% by weight of a combination of stearic acid and coconut oil fatty acid monoethanolamide.

6. A process for producing cleaning and disinfecting tablets corresponding to any of Claims 1 to 5, characterized in that at least two plasticizable mixtures containing incompatible substances are separately extruded in strand form, the separate strands are combined into a single strand and the single strands is cut up into tablets weighing from 20 to 200 g.

7. A process as claimed in Claim 6 in which a tablet comprising in its core a mixture containing dye or aggressive substances is prepared by extrusion in a coaxial screw extruder.

**Revendications**

1. Comprimé d'agent nettoyant et désinfectant pour les réservoirs d'eau des toilettes à chasse, ce comprimé comprenant des substances choisies parmi le groupe comprenant les agents tensio-actifs, les agents désinfectants, les agents de blanchiment, les sels, les acides, les agents complexants, les charges, les colorants, les substances odorantes, les régulateurs de rinçage et les plastifiants, ainsi qu'éventuellement d'autres substances habituellement utilisées dans de telles formulations, caractérisé en ce que:
a) le comprimé est constitué d'au moins deux mélanges plastifiables associés l'un avec l'autre,
b) au moins deux des mélanges contiennent des substances incompatibles l'une avec l'autre choisies, d'une part, parmi le groupe comprenant les substances dissociant du chlore actif, les acides et les agents complexants et, d'autre part, parmi le groupe comprenant les colorants et les parfums,
c) les mélanges ont une consistance identique ou semblable, et
d) on fabrique le comprimé par extrusion séparée des mélanges individuels, rassemblement des boudins en un seul boudin et découpage de ce boudin en morceaux pesant 20-200 g.

2. Comprimé d'agent nettoyant et désinfectant selon la revendication 1 que l'on prépare par extrusion au moyen d'une presse à vis sans fin coaxiale, de telle sorte qu'un mélange contenant un colorant ou des substances agressives soit situé dans le noyau du comprimé.

3. Comprimé d'agent désinfectant et nettoyant selon une des revendications 1 ou 2, dans lequel le rapport entre le diamètre et l'épaisseur est de 1 minimum.

4. Comprimé d'agent désinfectant et nettoyant selon une des revendications 1 à 3, constitué de deux mélanges plastifiables A et B ayant les compositions suivantes:
A: 15-60% en poids d'un alkyl-benzène-sulfonate
0-60% en poids de sels inorganiques
5-50% en poids d'agents désinfectants dissociant du chlore actif
3-20% en poids d'un agent plastifiant et/ou d'une substance odorante
0-25% en poids de substances actives réglant le rinçage
B: 15-60% en poids d'un alkyl-benzène-sulfonate
0-60% en poids de sels inorganiques
2-15% en poids d'un colorant
3-20% en poids d'un agent plastifiant et/ou d'une substance odorante
0-25% en poids de substances actives réglant le rinçage.

5. Comprimé d'agent désinfectant et nettoyant selon la revendication 4, ayant la composition suivante:

A: 15-60% en poids d'un alkyl-benzène-sulfonate

0-60% en poids de sels inorganiques

6-60% en poids d'agents désinfectants dissociant du chlore actif

3-12% en poids de plastifiants choisis parmi le groupe comprenant l'essence parfumée, l'huile de paraffine, le 1,2-propylène-glycol, l'huile de silicone, le phthalate de dibutyle, le mono-éthylène-glycol, les terpènes d'agrumes, le phthalate de diéthyle et leurs mélanges,

2-16% en poids d'une combinaison d'acide stéarique et de mono-éthanolamide d'acide gras de coco

B: 16-60% en poids d'un alkyl-benzène-sulfonate

0-60% en poids de sels inorganiques

2-16% en poids d'un colorant

3-12% en poids de plastifiants choisis parmi le groupe comprenant l'essence parfumée, l'huile de paraffine, le 1,2-propylène-glycol, l'huile de silicone, le phtalate de dibutyle, le mono-éthylène-glycol, les terpènes d'agrumes, le phtalate de diéthyle et leurs mélanges,

2-16% en poids d'une combinaison d'acide stéarique et de mono-éthanolamide d'acide gras de coco.

6. Procédé de fabrication de comprimés d'agents désinfectants et nettoyants selon une des revendications 1 à 6, caractérisé en ce qu'on extrude, sous forme de boudins, au moins deux mélanges plastifiables contenant chacun des substances mutuellement incompatibles, on rassemble les boudins individuels en un seul boudin et on découpe ce dernier en comprimés pesant 20-200 g.

7. Procédé selon la revendication 6, dans lequel, par extrusion au moyen d'une presse à vis sans fin coaxiale, on fabrique un comprimé comportant, dans son noyau, un mélange contenant un colorant ou des substances agressives.